# EUROPEAN PATENT SPECIFICATION

(11) **EP 4 067 871 B1**
(45) Date of publication and mention of the grant of the patent: **29.10.2025**
(21) Application number: 22153210.4
(22) Date of filing: 25.01.2022
(51) Int. Cl.: G01N 21/03, G01N 21/25, G02B 21/00, G02B 21/16, G02B 21/33, G02B 21/34, C12Q 1/6816, G01N 21/64

(54) **CONSTITUENT PART OF A MARKER**
BESTANDTEIL EINES MARKERS
PARTIE CONSTITUTIVE D'UN MARQUEUR

(30) Priority: 01.04.2021 WO PCT/EP2021/058785; 04.05.2021 WO PCT/EP2021/061754; 19.05.2021 WO PCT/EP2021/063310; 18.06.2021 WO PCT/EP2021/066645; 28.08.2021 WO PCT/EP2021/073819; 03.09.2021 WO PCT/EP2021/074412; 11.01.2022 EP 22150908
(43) Date of publication of application: 05.10.2022
(73) Proprietor: Leica Microsystems CMS GmbH, 35578 Wetzlar (DE)
(72) Inventor: Alsheimer, Soeren, 60320 Frankfurt (DE)
(74) Representative: Schaumburg und Partner Patentanwälte mbB

(56) References cited:
- US-A1- 2020 058 371
- US-A1- 2020 332 351
- MAKINO KOKI ET AL: "Color-Changing Fluorescent Barcode Based on Strand Displacement Reaction Enables Simple Multiplexed Labeling", JOURNAL OF THE AMERICAN CHEMICAL SOCIETY, vol. 144, no. 4, 20 January 2022 (2022-01-20), pages 1572 - 1579, XP055941768, ISSN: 0002-7863, DOI: 10.1021/jacs.1c09844
- DUNCOMBE TODD A ET AL: "Droplet barcoding: tracking mobile micro-reactors for high-throughput biology", CURRENT OPINION IN BIOTECHNOLOGY, LONDON, GB, vol. 60, 12 June 2019 (2019-06-12), pages 205 - 212, XP085930991, ISSN: 0958-1669, [retrieved on 20190612], DOI: 10.1016/J.COPBIO.2019.05.004

## Description

### Technical field

The invention relates to a constituent part of a marker and a method for assigning sequencing data to imaging data of biological samples embedded in a discrete entity comprising the constituent part.

### Background

Rare cells, like e.g. adult stem cells, circulating tumour cells and reactive immune cells (e.g. T-Cells, B-Cells, or NK-cells reactive to a certain antigen), are of great interest to basic and translational researchers. Reactive immune cells such as B-cell clones for instance that react to a certain pathogen, e.g. a virus, and can produce antibodies against a particular pathogen are of great value to generate urgently needed therapeutic antibodies. Similarly, reactive T-cells are sought after in the context of personalised medicine and the treatment of cancer and other diseases. Once a reactive T-cell is identified and isolated, the genetic sequence encoding the corresponding T-cell receptor displaying affinity against the target antigen can be cloned and used to generate genetically engineered T-cells such as CAR-T cells. Similarly, circulating tumour cells are expected to have great value for diagnosing cancer, predicting outcomes, managing therapies, and for the discovery of new cancer drugs and cell-based therapeutics. Suspensions of cells containing rare cells are typically derived from either a tissue sample by means of dissociation or from a liquid biopsy. The identification, analysis, and isolation of rare cells in these samples, particularly the analysis of these cells on the single cell level (single cell analysis, SCA) is therefore of great value for basic and translational research, diagnostic and therapeutic applications as well as in the context of bioprocessing and development and manufacturing of biologics and cellular therapeutics.

As the ability to identify and differentiate diverse cell types expands, the identification of cell types becomes more granular, i.e. the rare cell populations of interest are smaller and better defined. Thus, in order to find rare cells of interest a high (< 100k), very high (< 1M), or ultra-high (> 1M) number of cells typically needs to be analysed.

Recent progress in the fields of cell culture research has led to the advent of 3D cell culture, which is based on cultivating cells in three dimensions, for example, in suspension culture (scaffold-free techniques) or embedded in hydrogels and/or extracellular matrices (scaffold-based techniques). Hydrogels and extracellular matrices have been used extensively in conjunction with other elements for scaffold-based 3D cell culture. Cells and other elements can be efficiently embedded into discrete entities such as hydrogel beads by various means, cultivated in suspension, and imaged. Various forms of hydrogel beads including single-phase, multi-phase, mixed phase, hollow as well as solid core hydrogel beads with or without a shell can be manufactured using a variety of approaches including microfluidics, 3D printing, emulsification or electro-spraying. This allows cultivation of large numbers of cells, including rare cells, for analytical, diagnostic and therapeutic purposes in a 3D cell culture.

The cultivation of cells embedded in hydrogels (i.e. scaffold-based cell culture), which are kept in suspension, combines the benefits of scaffold-based cell culture with the benefits of suspension cell culture and is thus an attractive mode of cell culture for a wide range of applications. For many workflows that could be based on this type of cell culture, it would be required to be able to repeatably recognise individual discrete entities. For example, in case of handling a large number of single embedded cells, including rare cells, in a collective 3D cell culture, it is of great interest to be able to follow individual entities or cells, for example, over the course of an experiment. This would allow identification, analysis, and isolation especially of rare cells within the large number of cells. In addition, the analysis, for example by sequencing, of the genetic material of these individually embedded cells is of great interest. However, handling large numbers of entities and cells in a single vessel, whilst keeping track of each, is currently not possible. This is further complicated when considering that when carrying out different analyses, that may not all be based on optical information and they may be destructive. Therefore, it is desirable to be able to keep track of individual embedded cells and assign analysis data from different types of analyses to a particular embedded cell from a large number of pooled cells.

Makino et al. (2022, J. Am. Chem. Soc. 144, 1572-1579) discloses oligonucleotide constructs used for multiplex labelling, wherein the used oligonucleotide constructs all have the same sequence.

### Summary

The invention is defined by the appended claims.

It is an object to provide a plurality of constituent parts of a marker and a method, that enable keeping track of embedded biological samples between different types of analyses in a fast and efficient way.

The aforementioned object is achieved by the subject-matter of the independent claims. Advantageous embodiments are defined in the dependent claims and the following description.

A plurality of constituent parts of a marker is provided for marking or for identifying discrete entities comprising: a support structure; at least one first oligonucleotide connected to the support structure; at least a second oligonucleotide at least partially complementary to a part of the first oligonucleotide; and at least one label directly or indirectly connected to the second oligonucleotide, characterised in that at least a sequence of the complementary part between the first oligonucleotide and the second oligonucleotide is predetermined based on the particular label of the constituent part, wherein the predetermined sequence of each constituent part is a unique sequence.

The marker for marking the discrete entity is, in particular, an optically detectable pattern or structure comprising a plurality of the constituent parts, that can be read-out by means of a microscope, for example, and which allows to individually identify the discrete entity. In particular, the spatial and/or physical characteristics of the respective marker and its constituent parts in the discrete entity enable to have a large number of unique markers.

An oligonucleotide is, for example, a single stranded DNA or RNA molecule, that may be sequenced to determine its sequence of nucleotides. Complementary parts of oligonucleotides may hybridise or bind to each other. In particular, the second oligonucleotide may bind to the first oligonucleotide where both are complementary to each other. Preferably, the biological sample comprises at least one cell.

This enables marking the discrete entity with the embedded biological sample, in particular, with the plurality of the constituent parts. The discrete entity marked by constituent parts may be identified optically, but also by sequencing of the oligonucleotides of the constituent part. The constituent part enables relying not on a single way of identifying the discrete entity, but being able to identify the discrete entity in multiple independent ways.

Preferably, the discrete entity is comprised of a polymeric compound. The polymeric compound can be polymerised to form the discrete entity. In particular, the polymeric compound can form a hydrogel. The diameter of the discrete entities or hydrogel beads may be in the range of 10µm to 10mm. Particularly preferred ranges are 10µm to 100µm, 50µm to 250µm and 500µm to 5mm. This enables culturing of the biological sample in the discrete entity in scaffold-based suspension 3D cell culture, which combines the benefits of 3D suspension cell culture and scaffold-based cell culture.

For example, a method for optically identifying or recognising at least a target discrete entity with a biological sample and a marker, the marker comprising a plurality of constituent parts, from a plurality of discrete entities comprises the following steps: acquiring a first optical read-out of the marker of at least one discrete entity from the plurality of discrete entities, said discrete entity defining the target discrete entity; generating a first set of representations of the marker based on the first optical read-out of the marker of the target discrete entity and associating the first set of representations with the target discrete entity; acquiring a second optical read-out of the marker of at least one discrete entity from the plurality of discrete entities; generating a second set of representations of the marker based on the second optical read-out of the marker of the discrete entity; comparing the second set of representations to the first set of representations; recognising the discrete entity of the second optical read-out as the target discrete entity, when the second set of representations matches the first set of representations, in particular based on a measure of similarity and a statistical confidence score.

For further examples of a discrete entity comprising a marker and for further examples of a generalised method for recognising a discrete entity comprising a marker, reference is made to the applications PCT/EP2021/058785 and PCT/EP2021/061754,
In particular, when identifying a discrete entity and/or determining a match of a first image of at least one discrete entity with at least a second image of the at least one discrete entity the steps for generating the representations may comprise: determining vectors from at least one reference item to at least some of the constituent parts of the marker, in particular determining vectors from at least one reference item to the centre of mass of at least some of the respective constituent parts of the marker, determining for the vectors at least one value of a property of the vectors, and generating the representation of the marker based on the frequency of the values of the property. Further, the representation may be generated as a hash value of the values of the property, in particular, a frequency of the values of the property. This is further detailed in the application EP22150908, .

Preferably, the label comprises at least a first fluorophore. Thus, the label comprises at least one fluorophore or a fluorescent molecule that may be optically detected. This enables particular easy optical detection of the constituent part of the marker.

Preferably, the support structure is a microbead, particularly a polystyrene microbead, a DNA origami-based structure or a nanoruler, especially as described in the application PCT/EP2021/074412, .

DNA origami comprises oligonucleotides in particular, the nanoruler may comprise DNA origami. The microbead may have a diameter in the range of 50nm to 500nm, 250nm-1µm, or 0.5µm-10µm.

Preferably, the label comprises at least a second fluorophore. Thus, the label is at least two fluorescent molecules, which may be directly or indirectly connected to the second oligonucleotide. This enables a large variety of constituent parts.

It is preferable, that the first fluorophore and the second fluorophore differ in their optical properties. The optical properties may, in particular, be fluorescent properties, for example, fluorescent wavelength, excitation wavelength or fluorescence lifetime. Thus, labels of different constituent parts may differ from each other in their number of fluorophores and in the optical or fluorescent properties of the individual fluorophores. This enables a large variety of constituent parts and a particularly precise optical identification of the constituent part.

Specifically, the predetermined sequence is based on properties of the fluorophore, for example, the excitation or emission wavelength, and/or on the number of fluorophores of the label. This enables particularly precise identification of the constituent part by sequencing.

Preferably, the first fluorophore and/or the second fluorophore are each connected to distinct and/or unique parts of the second oligonucleotide, for example, by an oligonucleotide linker complementary to the respective unique part of the second oligonucleotide. This enables easy assembly of the constituent part.

Preferably, the part of the first oligonucleotide partially complementary to the second oligonucleotide and/or the part of the second oligonucleotide partially complementary to the first oligonucleotide is cleavable from the first oligonucleotide or the second oligonucleotide, respectively. For example, the first oligonucleotide and/or the second oligonucleotide may comprise a cleavage site, for cleaving the part of the first oligonucleotide partially complementary to the second oligonucleotide and/or the part of the second oligonucleotide partially complementary to the first oligonucleotide from the first oligonucleotide or the second oligonucleotide, respectively. The cleavage site may be a restriction site, which may be cut by a corresponding restriction enzyme.

Preferably, the first fluorophore is connected to the second oligonucleotide by a third oligonucleotide partially complementary to a first part of the second oligonucleotide. This enables easy attachment of the second fluorophore to the second oligonucleotide.

Preferably, the second fluorophore is connected to the second oligonucleotide by a fourth oligonucleotide partially complementary to a second part of the second oligonucleotide. This enables easy attachment of the second fluorophore to the second oligonucleotide. In particular, the first and second part of the second oligonucleotide have different sequences. This enables specific attachment of each fluorophore to the second oligonucleotide.

In another aspect, a method is provided for assigning sequencing data to imaging data of embedded biological samples, the method comprising the following steps: providing a plurality of discrete entities each comprising a biological sample and a plurality of constituent parts, according to one of the preceding claims, the plurality of constituent parts forming a marker of the discrete entity, wherein the constituent parts are grouped in at least a first set of constituent parts and a second set of constituent parts, and for each of the sets of constituent parts, the constituent parts comprise a unique label and a unique predetermined complementary part between the first and the second oligonucleotide; imaging the discrete entities to generate imaging data of the corresponding biological samples and markers; determining a frequency of all the unique labels in the imaging data for each discrete entity; individually disintegrating the discrete entities to release the corresponding biological sample and constituent parts of the marker and, in particular, releasing the genetic material of the biological sample and the constituent parts of the marker; individually sequencing the corresponding biological samples, in particular the genetic material, and at least the corresponding predetermined complementary parts between the first and the second oligonucleotide of the constituent parts of the marker to generate sequencing data, determining a frequency of all the unique predetermined complementary parts in the sequencing data for each discrete entity; and assigning or matching for a particular discrete entity, the sequencing data of the biological sample to the imaging data of the biological sample based on the frequency of the unique labels and the frequency of the unique predetermined complementary parts.

The unique label of the constituent parts may have a particular number of fluorophores and each fluorophore may have different fluorescent properties. For each set of constituent parts, the labels and the predetermined sequences are the same. Between sets of constituent parts, the labels differ in their number of fluorophores and/or their fluorescent properties and the predetermined sequences differ in their nucleotide sequence.

The imaging of the of the discrete entities is, in particular, fluorescent imaging, including projecting excitation light on the discrete entities to excite fluorophore of each label and image the emission light of the label.

The sequencing is, in particular, quantitative. This means, that the relative or absolute amounts of genetic material with a particular sequence may be determined. Thus, the predetermined sequences may be quantitatively determined.

When determining the frequency of the predetermined complementary parts, this means the number of times the unique sequence was found by sequencing is determined.

When assigning or matching, the sequencing data of the biological sample to the imaging data of the biological sample based on the frequency of the unique labels and the frequency of the unique predetermined complementary parts, this means the frequency of the particular sequence and the frequency of the particular labels are compared and assigned when they correlate. In particular, if the frequency of the constituent parts correlates with the frequency of the predetermined sequences, the sequencing data of the particular discrete entity is assigned to the imaging data. Since the number of constituent parts in each set of constituent parts of a particular discrete entity determines the frequency of the constituent parts determined in the imaging data and the frequency of the predetermined sequences, both frequencies for the particular discrete entity correlate with each other. For example, in the imaging data of a discrete entity five constituent parts of a particular set of constituent parts may be identified and the sequencing data for that discrete entity would have a proportional amount of the unique predetermined sequence of the five constituent parts. This enables assigning imaging data of a particular discrete entity to the sequencing data of that particular discrete entity.

Preferably, the properties of the fluorophores include the excitation wavelength and fluorescent wavelength. This enables particularly easy detection of the marker.

Preferably, the discrete entity is comprised of a polymeric compound, in particular a hydrogel. This enables particularly easy suspension culturing of the biological sample.

Preferably, the discrete entities are imaged by means of a microscope, in particular, a light sheet microscope. This enables particularly precise imaging of the marker and the biological sample.

The method has the same advantages as the constituent part described above. In particular, the method may be supplemented using the features of the dependent claims directed at the constituent part.

Further features and advantages of the invention result from the claims and the following description of preferred embodiments, which are described with reference to the accompanying drawings.

### Short Description of the Figures

Hereinafter, specific embodiments are described referring to the drawings, wherein:
- Fig. 1: shows a schematic view of a constituent part of a marker,
- Fig. 2: shows a schematic view of a discrete entity with a marker comprising constituent parts according to Figure 1,
- Fig. 3: shows a schematic view of further embodiments of the constituent part,
- Fig. 4: shows a schematic overview of imaging data and sequencing data generated from the discrete entity,
- Fig. 5: shows a schematic view of a constituent part with a label comprising multiple fluorophores, and
- Fig. 6: shows a flow chart for a method for assigning sequencing data to imaging data of biological samples.

### Detailed Description

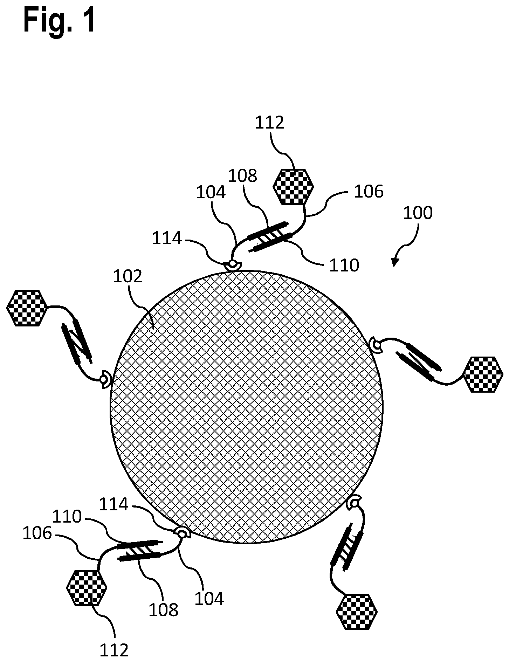

Figure 1 shows a schematic view of a constituent part 100 of a marker. The constituent part 100 comprises a microbead 102 to which is attached a plurality of first oligonucleotides 104. The microbead 102 has a diameter in the range of 50 nm to 500 nm, 250nm to 1µm, or 0.5µm to 10µm.

Further, the constituent part 100 comprises a plurality of second oligonucleotides 106, which are each attached to one of the first oligonucleotides 104. Specifically, the first oligonucleotides 104 and the second oligonucleotides 106 are partially complementary to each other. This means, that a part 108 of the sequence of the first oligonucleotide 104 and a part 110 of the sequence of the second oligonucleotides 106 are complementary and therefore bind specifically to each other. Thus, the parts 108, 110 may hybridise with each other. The first oligonucleotide 106, in turn, may be attached to the microbead 102 by an (affinity) linker 114 or by means of covalent coupling, which may be mediated by a bioconjugate or by coupling chemistries like NHS or click chemistries using alkynes or azides, for example strain-promoted azide-alkyne cycloaddition or strain-promoted alkyne-nitrone addition. The affinity linked 114 may be a streptavidin / biotin link, for example, where the first oligonucleotide 106 comprises one of streptavidin or biotin and the microbead 102 comprises the other one of streptavidin or biotin. Thus, the specific binding of the part 108 of the first oligonucleotide 104 to the part 110 of the second oligonucleotide 106 and the first oligonucleotide 104 to the microbead 102 enables particularly easy assembly of the constituent part 100. The individual oligonucleotides may be single-stranded DNA or RNA. When DNA origami-based structures, such as nanorulers, are used as a support structure, the linker 114 may be a staple strand configured to bind the DNA origami-based structure at a predetermined position.

Further, a label is connected to the second oligonucleotide 106. The label specifically is at least one fluorophore 112. The fluorophore 112 may be optically detected, for example, the fluorescence of the fluorophores may be detected by means of a microscope.

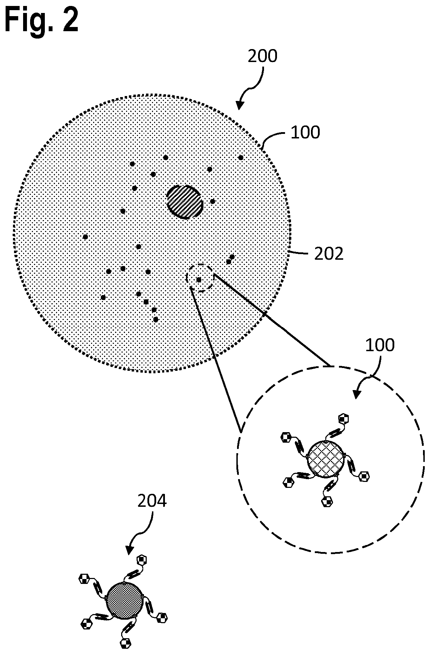

Figure 2 shows a schematic view of a hydrogel bead 200, as an example of a discrete entity, with a marker comprising a plurality of the constituent parts 100. The hydrogel bead 200 further contains a biological sample 202.

The hydrogel bead 200 is made of a polymeric compound, in particular, a polymeric compound that forms a hydrogel and/or that is substantially transparent. The polymeric compound may be of natural or synthetic origin, including for example, agarose, alginate, chitosan, hyaluronan, dextran, collagen and fibrin as well as poly(ethylene glycol), poly(hydroxyethyl methacrylate), poly(vinyl alcohol) and poly(caprolactone). The hydrogel bead 200 may be made of a single or several different polymeric compounds.

The hydrogel bead 200 may comprise several sections such as an inner core and an outer layer around the core. Each of the sections can be made of a particular polymeric compound. The sections of the hydrogel bead 200 can each have different properties. These properties include physicochemical properties such as Young's modulus, refractive index, and chemical composition and functionalisation.

The shape of the hydrogel bead 200 is spherical. Alternatively, the hydrogel bead 200 may have a different shape such as a spheroid. The diameter of the hydrogel bead 200 may be in the range of 10µm to 10mm. Particularly preferred ranges are 10µm to 100µm, 50µm to 250µm and 500µm to 5mm.

The hydrogel bead 200 can be formed, for example, by electrospray, emulsification, lithography, 3D printing and microfluidic approaches. During formation of the hydrogel bead 200 and before polymerisation of the hydrogel, the constituent parts 100 may be added to the hydrogel bead 200.

The constituent parts 100 are included and randomly dispersed in the hydrogel bead 200 during the formation of the hydrogel bead 200. After the formation of the hydrogel bead 200, the parts 100 are set in place in the hydrogel bead 200. This means they do not change their location in the hydrogel bead 200 once the hydrogel bead 200 is formed, resulting in substantially stable discrete entities or hydrogel beads 200.

The hydrogel bead 200 includes the biological sample 202, for example, a cell or a cluster of cells. The cell may be a eukaryotic or a prokaryotic cell, including archaea, bacteria, plant, mammalian, non-mammalian cells and fungi. The cluster of cells may be a spheroid, a tumoroid or an organoid. In addition, the biological samples 202 can be a co-culture of different cell types, bacteria, viruses, prions, cellular pathogens and/or multicellular parasites.

The marker comprises a plurality of the constituent parts 100 of the hydrogel bead 200. For example, the number of constituent parts 100 characterising the marker of the hydrogel bead 200 may be used to repeatedly recognise the hydrogel bead 200 in images of the hydrogel bead 200.

In an alternative embodiment, there may be several sets of constituent parts in the hydrogel bead 200. For example, a first set of constituent parts may comprise the plurality of the constituent parts 100. A second set of constituent parts may comprise a plurality of constituent parts 204. The second set may differ from the first set only in that the constituent parts 204 comprise a fluorophore with different fluorescent properties, such as emission wavelength, compared to the fluorophore 112 of the constituent part 100. In addition or alternatively, more than one fluorophore may be connected to the second oligonucleotide 106. Both sets of constituent parts 100, 204 may be used to generate the marker of a hydrogel bead. By varying the number of constituent parts 100, 204 and their ratio in different hydrogel beads, different markers with unique frequencies of the constituent parts 100, 204 may be generated. This enables recognising or identifying the different hydrogel beads according to their unique marker when imaging them repeatedly.

In any case, the constituent parts 100 of the marker are optically detectable, for example, as an intensity object by means of a microscope. Thus, the marker is optically detectable. The hydrogel bead 200 is required to be transparent, at least to an extent that allows the sample 202 and the parts 100 of the marker to be optically detected.

Moreover, the oligonucleotides, in particular, the parts 108, 110 may comprise a predetermined nucleotide sequence based on the particular fluorophore or fluorophores of the label. Specifically, the predetermined sequence may be based on fluorescent properties of the fluorophores, such as excitation wavelength or emission wavelength, or the number of fluorophores comprised by the label. Thus, the predetermined sequence is unique to a particular combination of fluorophores and/or their particular fluorescent properties. Thus, each set of constituent parts may have a unique predetermined sequence. This enables identification by sequencing of the genetic material of a particular constituent part 100 as belonging to a particular set of constituent parts with its the associated fluorescent properties of the fluorophores of the particular set.

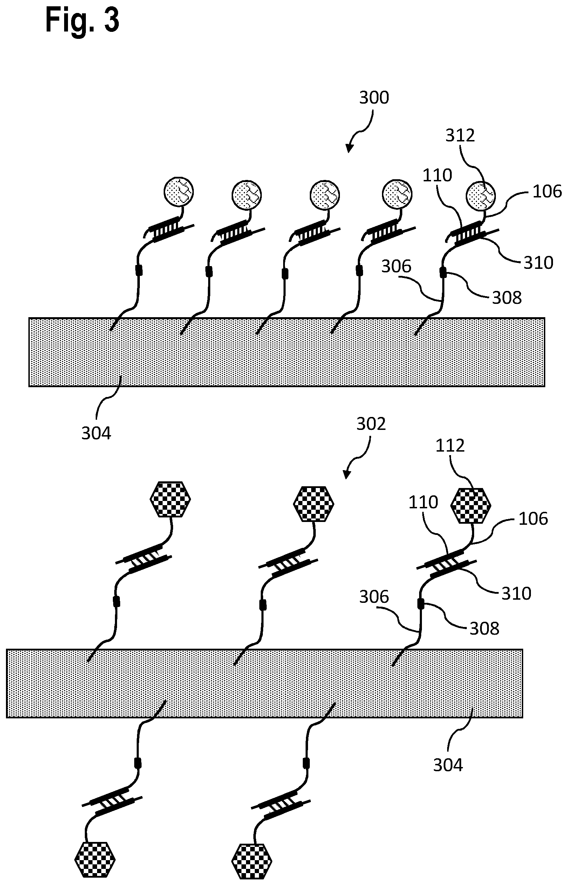

Figure 3 shows a schematic view of alternative constituent parts 300, 302. The constituent parts 300, 302 both have a support structure comprising DNA origami 304. The constituent parts 300, 302 may, for example, be embodied as described in the application PCT/EP2021/074412.

Further, the constituent parts 300, 302 comprise a first oligonucleotide 306 with a cleavage site 308. At the cleavage site 308 the oligonucleotides may be cut with a restriction enzyme. Moreover, the constituent parts 300, 302 comprise the second oligonucleotide 106, as described above. In an alternative embodiment the second oligonucleotides 106 may also comprise a cleavage site.

Moreover, similar to what is described above, a part 310 of the first oligonucleotide 306 and the part 110 of the second oligonucleotides 106 are complementary and therefore bind specifically to each other. This enables attaching the second oligonucleotide 106 to the first oligonucleotide 306. The fluorophore 112 is attached to the second oligonucleotide 106. The constituent part 300 comprises a fluorophore 312, which has different fluorescent properties to the fluorophore 112.

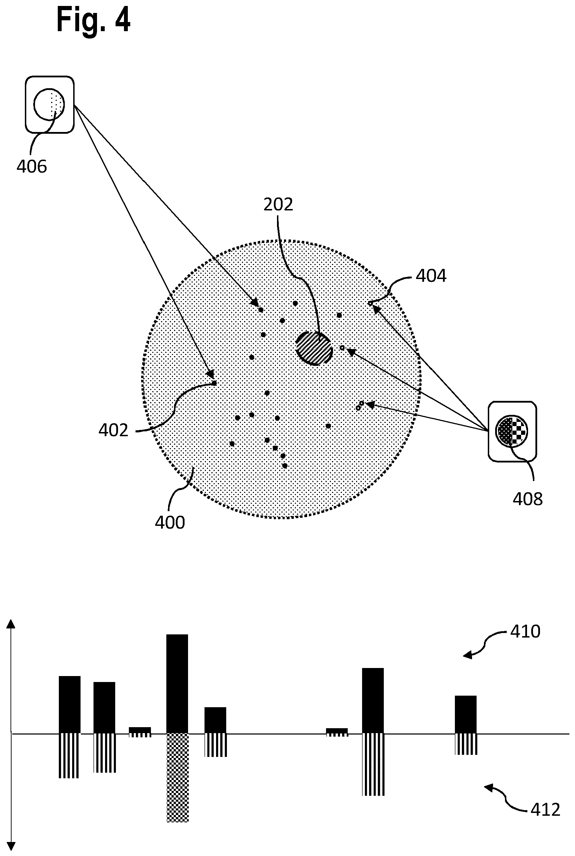

Figure 4 shows a schematic overview of imaging data and sequencing data generated from a hydrogel bead 400. The hydrogel bead 400 comprises a plurality of e.g. eight different sets of constituent parts, of which only two sets of constituent parts are shown in Figure 4, i.e. a first set of constituent parts 402 and a second set of constituent parts 404. The constituent parts 402 comprise a fluorophore 406 and the constituent parts 404 comprise a fluorophore 408. The fluorophores 406, 408 have different fluorescent properties from each other, for example, their excitation wavelength and/or their emission wavelength differ. In addition, as described above, each set of constituent parts, in particular the complementary part of the first and second oligonucleotides, has a unique predetermined sequence.

Thus, the hydrogel bead 400 may be imaged, for example to study the biological sample 202, and the frequency of the labels of the constituent parts 400, 402 of the first and second set of constituent parts may be determined. This means, the number of labels of the constituent parts 400, 402 may be determined from the image of the hydrogel bead 400. The frequency of the labels is indicated by reference sign 410.

Subsequently, for example, when determining the genetic content of the biological sample 202 of the hydrogel bead 400 by sequencing, the genetic content of the oligonucleotides, in particular the predetermined sequences, of the constituent parts of the marker of the hydrogel bead 400 may equally be determined by sequencing. The sequencing is, in particular, quantitative. This means, that the relative or absolute amounts of genetic material of a particular sequence may be determined. Thus, the predetermined sequences may be quantitatively determined. The frequency of the predetermined sequences is indicated by reference sign 412.

A comparison of the frequency of the predetermined sequences 412 with the frequency of the labels 410 enables assigning or matching images of the hydrogel bead 400 including the biological sample 202 to the sequencing data generated when sequencing the genetic content of the biological sample 202. In particular, when the number of times the labels were detected correlates with the number of times the predetermined sequences were detected, the images are matched. In Figure 4 the frequencies 410, 412 are exemplarily shown for the eight sets of constituent parts, each bar representing one of the sets of constituent parts, in particular, each bar represents the number of labels or number of predetermined sequences in a particular one of the sets of constituent parts.

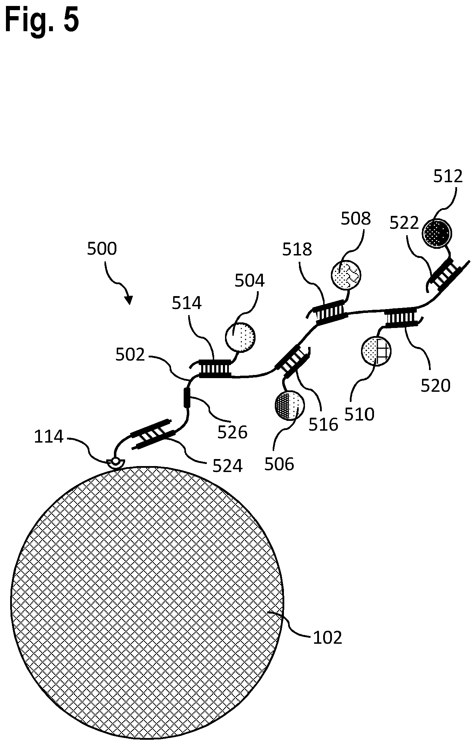

Figure 5 shows a schematic view of a constituent part 500 with a label comprising multiple fluorophores. Attached to a second oligonucleotide 502 are a first fluorophore 504, a second fluorophore 506, a third fluorophore 508, a fourth fluorophore 510 and a fifth fluorophore 512. Each of the fluorophores 504, 506, 508, 510, 512 is attached to the second oligonucleotide 502 by a respective third oligonucleotide 514, fourth oligonucleotide 516, fifth oligonucleotide 518, sixth oligonucleotide 520 or seventh oligonucleotide 522. Each of the oligonucleotides 514, 516, 518, 520, 522 is partially hybridised to a complementary sequence on the second oligonucleotide 502. The complementary sequence is unique for each of the oligonucleotides 514, 516, 518, 520, 522, such that the fluorophores 504, 506, 508, 510, 512 may be specifically attached to the second oligonucleotide 502.

Each of the fluorophores 504, 506, 508, 510, 512 differs from the other fluorophores 504, 506, 508, 510, 512 in at least one fluorescent property. For example, the fluorophores 504, 506, 508, 510, 512 may differ in their emission wavelength and/or their excitation wavelength. This enables generating a large number of different sets of constituent parts, with each set comprising a particular combination of fluorophores with particular fluorescent properties.

As described before, the second oligonucleotide 502 further comprises a part 524 that is complementary to part of a first oligonucleotide that is linked to a support structure such as the microbead 102 or the DNA-origami 304. The part 524 comprises a predetermined nucleotide sequence based on the fluorescent properties of the fluorophores 504, 506, 508, 510, 512 attached to the second oligonucleotide 502. This means that based on the unique predetermined sequence the fluorophores 504, 506, 508, 510, 512 attached to the second oligonucleotide 502 can be unambiguously identified. This enables identification of the fluorophores of a particular constituent part by sequencing of the predetermined sequence of that particular constituent part.

In order to facilitate the sequencing of the part 524 with the predetermined sequence, a cleavage site 526 or multiple cleavage sites may be provided, as described above. The cleavage site 526 allows cutting the part 524 from the second oligonucleotide 502 with a suitable restriction enzyme and isolating it prior to sequencing.

In order to facilitate the sequencing of the part 524 with the predetermined sequence, a landing site or complementary sequence to a universal oligonucleotide may be provided to enable sequencing of different predetermined sequences using the same primer oligonucleotide.

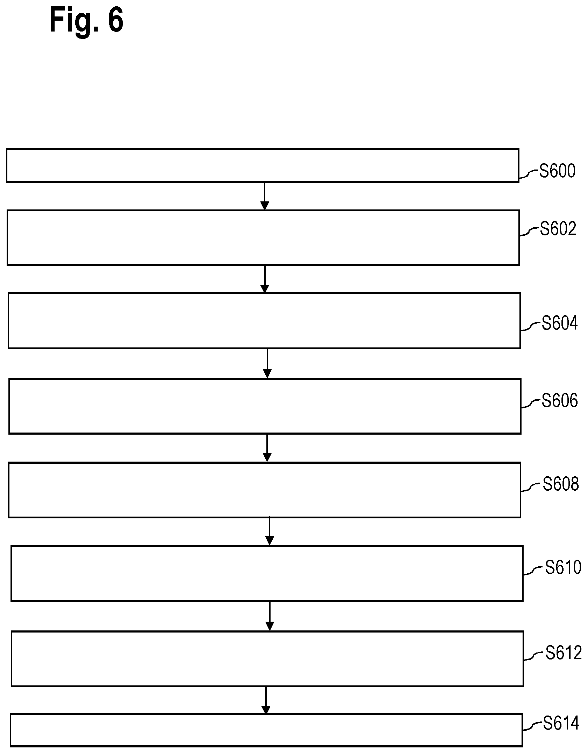

Figure 6 shows a flow chart for a method for assigning sequencing data to imaging data of biological samples by means of constituent parts of a marker. The method starts with step S600. In step S602 hydrogel beads 100, each comprising one of the biological samples 202 and at least a first set of constituent parts 100 of a marker and a second set of constituent parts, are imaged. The hydrogel beads 100 may be imaged by means of an imaging device such as a microscope or an imaging flow cytometer. Each of the sets of constituent parts comprises constituent parts with the same unique fluorophore or fluorophores and a corresponding unique predetermined sequence of the complementary part between the first oligonucleotide and the second oligonucleotide of the constituent parts.

Preferably, the number of constituent parts in each set varies between each of the hydrogel beads. This enables providing the hydrogel beads with unique markers and subsequent identification or recognition of individual hydrogel beads based on their number of constituent parts.

In case the method is to be carried out on a large number of hydrogel beads, a plurality of sets of constituent parts, with each set having a unique fluorophore or fluorophores and a unique predetermined sequence may be used as a marker for each hydrogel bead 100. This enables generating a larger variety of markers in order to provide unique markers in each of the large number of hydrogel beads.

Each hydrogel bead 100 may be imaged several times, for example, during a time course experiment, iterative staining process, or during sequential assays, each image showing at least the biological sample and the constituent parts of the marker of the respective hydrogel bead.

The images may be three-dimensional image is, for example, a z-stack, or image stack, comprising a plurality of two-dimensional images of the hydrogel beads, in particular, of parallel images. Such a stack of images enables generating the three-dimensional image of the respective hydrogel bead.

In step S604 for each image a frequency is determined of all the constituent parts of the particular imaged hydrogel bead. This means that the number of constituent parts for each of the sets of constituent parts are determined from the image data.

In order to determine the constituent parts in the image data, feature extraction may be carried out on the image data of the images generated in step S602. Feature extraction is typically performed by feeding n-dimensional image data of the first and second image through a suitable image processing pipeline or algorithm. Such an image processing pipeline may include background removal, compression, filtering, denoising, enhancement, reconstruction, correction, deconvolution, multi-view deconvolution, multi-view registration, multi-view fusion, and include an image segmentation step, which generates a set of segmented features of the discrete entity, as well as a feature classification step. The result of image segmentation and/or feature classification is typically a segmented virtual discrete entity, its centre of mass, the identified constituent parts of the marker, for example based on their fluorescent properties, as well as features belonging to the biological sample. Feature classification algorithms based on classical approaches, e.g. filtering for size, colour, shape, etc., as well as machine or deep learning based approaches may be used to reliably classify features into one of the aforementioned categories, i.e. as belonging to the discrete entity, the marker, or the biological sample.

In step S606, the hydrogel beads are individually disintegrated, for example enzymatically, in order to release the embedded biological sample and constituent parts of each of the hydrogel beads. In particular, the genetic material is released of the biological sample and the constituent parts of the marker of each of the hydrogel bead. The disintegration is performed individually such as to keep the genetic material of each of the hydrogel beads separate from each other. Specifically, the genetic material is the genomic DNA of the biological sample, RNA content of the biological sample and/or extra-chromosomal DNA, as well as the oligonucleotides, in particular the predetermined sequence, of the constituent parts of the marker of a particular one of the hydrogel beads. The disintegration may include cutting the first and/or second oligonucleotides of the constituent parts at cleavage sites. Further, the step may include extraction of the genomic material.

In step S608, the genetic material released from each hydrogel bead in step S606 is individually sequenced. This means, that for each hydrogel bead, the genetic material is sequenced separate from the other hydrogel beads. In particular, the genetic material is sequenced quantitatively. This means, that the relative or absolute amounts of genetic material of a particular sequence may be determined. First, second, third and fourth generation next generation sequencing methods are suited to perform the sequencing task. These methods include but are not limited to: Single-molecule realtime sequencing, Ion semiconductor, Pyrosequencing, Sequencing by synthesis, Combinatorial probe anchor synthesis, Sequencing by ligation, Nanopore Sequencing, GenapSys Sequencing.

In step S610, for each hydrogel bead a frequency is determined of all predetermined sequences based on the quantitative sequencing data generated for the particular hydrogel bead in step S608. This means that the number of predetermined sequences for each of the unique predetermined sequences of the sets of constituent parts are determined.

In step S612, for each particular hydrogel bead, the sequencing data of the hydrogel bead is assigned or matched to the images of the hydrogel bead based on the frequency of the predetermined sequences of step S610 and the frequency of the constituent parts of step S606. In particular, if the frequency of the constituent parts correlates with the frequency of the predetermined sequences, the sequencing data of the particular hydrogel bead is assigned to the imaging data. Since the number of constituent parts in each set of constituent parts of a particular hydrogel bead determines the frequency of the constituent parts determined in the imaging data and the frequency of the predetermined sequences, both frequencies for the particular hydrogel bead correlate with each other. For example, in the imaging data of a hydrogel bead five constituent parts of a particular set of constituent parts may be identified and the sequencing data for that hydrogel bead would have a proportional amount of the unique predetermined sequence of the five constituent parts. This enables assigning imaging data of a particular hydrogel bead to the sequencing data of that particular hydrogel bead. Due to this, the hydrogel beads do not have to be kept individually when carrying out the method, instead, the hydrogel beads may be mixed together between steps and between taking images in step S602. The method ends in step S614.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100, 204, 300, 302, 402, 404, 500: Constituent part of a marker
- 102: Microbead
- 104, 306: First oligonucleotide
- 106: Second oligonucleotide
- 108, 310: Part of first oligonucleotide
- 110: Part of second oligonucleotide
- 112, 406, 408, 504, 506, 508, 510, 512: Fluorophore
- 114: Affinity linker or covalent linker
- 200, 400: Hydrogel bead
- 202: Biological sample
- 304: DNA-origami
- 308, 526: Cleavage site
- 410: Frequency of labels
- 412: Frequency of unique predetermined sequences
- 514: Third oligonucleotide
- 516: Fourth oligonucleotide
- 518: Fifth oligonucleotide
- 520: Sixth oligonucleotide
- 522: Seventh oligonucleotide

## Claims

1. A plurality of constituent parts (100, 204, 300, 302, 402, 404, 500) of a marker for marking discrete entities (200, 400), each constituent part comprising:
a support structure (102, 304),
at least one first oligonucleotide (104, 306) connected to the support structure (102, 304),
at least a second oligonucleotide (106) at least partially complementary to a part (108, 310) of the first oligonucleotide (104, 306), and
at least one label (112, 406, 408, 504, 506, 508, 510, 512) connected to the second oligonucleotide (106), **characterised in that**
at least a sequence of the complementary part between the first oligonucleotide and the second oligonucleotide is predetermined based on the particular label of the constituent part, wherein the predetermined sequence of each constituent part is a unique sequence.

2. The constituent part according to claim 1, wherein the label (112) comprises at least a first fluorophore (504).

3. The constituent part according to one of the preceding claims, wherein the support structure (102, 304) is a microbead (102), a DNA origami-based structure (304) or a nanoruler.

4. The constituent part according to one of the preceding claims, wherein the label (112) comprises at least a second fluorophore (506).

5. The constituent part according to claim 4, wherein the first fluorophore (504) and the second fluorophore (506) differ in their optical properties.

6. The constituent part according to one of the claims 2 to 5, wherein the first fluorophore (504) and/or the second fluorophore (506) are each connected to distinct parts of the second oligonucleotide (106).

7. The constituent part according to one of the preceding claims, wherein the part (108, 310) of the first oligonucleotide (104, 306) partially complementary to the second oligonucleotide (106) and/or the part (110) of the second oligonucleotide (106) partially complementary to the first oligonucleotide (104, 306) is cleavable from the first oligonucleotide (104, 306) or the second oligonucleotide (106), respectively.

8. The constituent part according to one of the preceding claims, wherein the first fluorophore (104, 306) is connected to the second oligonucleotide (106) by a third oligonucleotide (514) partially complementary to a first part of the second oligonucleotide (106).

9. The constituent part according to one of the claims 4 to 8, wherein the second fluorophore (506) is connected to the second oligonucleotide (106) by a fourth oligonucleotide (516) partially complementary to a second part of the second oligonucleotide (106).

10. A method for assigning sequencing data to imaging data of biological samples (202), the method comprising the following steps;
providing a plurality of discrete entities (200, 400) each comprising a biological sample (202) and a plurality of constituent parts (100, 204, 300, 302, 402, 404, 500), according to one of the preceding claims, the constituent parts forming a marker of the discrete entity (200, 400), wherein the constituent parts are grouped in at least a first set of constituent parts and a second set of constituent parts, and for each of the sets of constituent parts, the constituent parts comprise a unique label and a unique predetermined complementary part between the first and the second oligonucleotide,
imaging the discrete entities (200, 400) to generate imaging data of the corresponding biological samples (202) and markers,
determining a frequency (410) of all the unique labels in the imaging data for each discrete entity (200, 400),
individually disintegrating the discrete entities (200, 400) to release the corresponding biological sample (202) and constituent parts of the marker,
individually sequencing the corresponding biological samples (202) and at least the corresponding predetermined complementary parts between the first oligonucleotide and the second oligonucleotide of the constituent parts of the marker to generate sequencing data,
determining a frequency (412) of all the unique predetermined complementary parts in the sequencing data for each discrete entity (200, 400), and
assigning for a particular discrete entity (200, 400), the sequencing data of the biological sample to the imaging data of the biological sample (202) based on the frequency of the unique labels and the frequency of the unique predetermined complementary parts.

11. The method according to claim 10, wherein properties of the fluorophores (112, 406, 408, 504, 506, 508, 510, 512) include the excitation wavelength and fluorescent wavelength.

12. The method according to one of the claims 10 and 11, wherein the discrete entity is comprised of a polymeric compound, in particular a hydrogel.

13. The method according to one of the claims 10 to 12, wherein the discrete entities (200, 400) are imaged by means of a microscope.

14. The method according to one of the claims 10 to 13, wherein the biological sample (202) comprises at least one cell.

## Patentansprüche

1. Vielzahl von Bestandteilen (100, 204, 300, 302, 402, 404, 500) eines Markers zum Markieren diskreter Einheiten (200, 400), wobei jeder Bestandteil Folgendes umfasst:
eine Stützstruktur (102, 304),
mindestens ein erstes Oligonukleotid (104, 306), das mit der Stützstruktur (102, 304) verbunden ist,
mindestens ein zweites Oligonukleotid (106), das mindestens teilweise komplementär zu einem Teil (108, 310) des ersten Oligonukleotids (104, 306) ist, und
mindestens ein Label (112, 406, 408, 504, 506, 508, 510, 512), die mit dem zweiten Oligonukleotid (106) verbunden ist, **dadurch gekennzeichnet, dass**
mindestens eine Sequenz des komplementären Teils zwischen dem ersten Oligonukleotid und dem zweiten Oligonukleotid basierend auf dem besonderen Label des Bestandteils vorbestimmt ist, wobei die vorbestimmte Sequenz jedes Bestandteils eine eindeutige Sequenz ist.

2. Bestandteil nach Anspruch 1, wobei das Label (112) mindestens ein erstes Fluorophor (504) umfasst.

3. Bestandteil nach einem der vorstehenden Ansprüche, wobei die Stützstruktur (102, 304) ein Mikrokügelchen (102), eine DNA-Origami-basierte Struktur (304) oder ein Nanoruler ist.

4. Bestandteil nach einem der vorstehenden Ansprüche, wobei das Label (112) mindestens ein zweites Fluorophor (506) umfasst.

5. Bestandteil nach Anspruch 4, wobei sich das erste Fluorophor (504) und das zweite Fluorophor (506) in ihren optischen Eigenschaften unterscheiden.

6. Bestandteil nach einem der Ansprüche 2 bis 5, wobei das erste Fluorophor (504) und/oder das zweite Fluorophor (506) jeweils mit unterschiedlichen Teilen des zweiten Oligonukleotids (106) verbunden sind.

7. Bestandteil nach einem der vorstehenden Ansprüche, wobei der Teil (108, 310) des ersten Oligonukleotids (104, 306), der teilweise komplementär zum zweiten Oligonukleotid (106) ist, und/oder der Teil (110) des zweiten Oligonukleotids (106), der teilweise komplementär zum ersten Oligonukleotid (104, 306) ist, vom ersten Oligonukleotid (104, 306) beziehungsweise vom zweiten Oligonukleotid (106) abspaltbar ist.

8. Bestandteil nach einem der vorstehenden Ansprüche, wobei das erste Fluorophor (104, 306) über ein drittes Oligonukleotid (514), das teilweise komplementär zu einem ersten Teil des zweiten Oligonukleotids (106) ist, mit dem zweiten Oligonukleotid (106) verbunden ist.

9. Bestandteil nach einem der Ansprüche 4 bis 8, wobei das zweite Fluorophor (506) über ein viertes Oligonukleotid (516), das teilweise komplementär zu einem zweiten Teil des zweiten Oligonukleotids (106) ist, mit dem zweiten Oligonukleotid (106) verbunden ist.

10. Verfahren zum Zuordnen von Sequenzierungsdaten zu Bilddaten biologischer Proben (202), wobei das Verfahren die folgenden Schritte umfasst;
Bereitstellen einer Vielzahl von diskreten Einheiten (200, 400), die jeweils eine biologische Probe (202) und eine Vielzahl von Bestandteilen (100, 204, 300, 302, 402, 404, 500) umfassen, nach einem der vorstehenden Ansprüche, wobei die Bestandteile einen Marker der diskreten Einheit (200, 400) bilden, wobei die Bestandteile in mindestens einem ersten Satz von Bestandteilen und einem zweiten Satz von Bestandteilen gruppiert sind und für jeden der Sätze von Bestandteilen die Bestandteile ein eindeutiges Label und ein eindeutiges vorbestimmtes komplementäres Teil zwischen dem ersten und dem zweiten Oligonukleotid umfassen,
Abbilden der diskreten Einheiten (200, 400), um Abbildungsdaten der entsprechenden biologischen Proben (202) und Marker zu erzeugen,
Bestimmen einer Häufigkeit (410) aller eindeutigen Labels in den Bilddaten für jede diskrete Einheit (200, 400),
individuelles Auflösen der diskreten Einheiten (200, 400), um die entsprechende biologische Probe (202) und Bestandteile des Markers freizusetzen,
individuelles Sequenzieren der entsprechenden biologischen Proben (202) und mindestens der entsprechenden vorbestimmten komplementären Teile zwischen dem ersten Oligonukleotid und dem zweiten Oligonukleotid der Bestandteile des Markers, um Sequenzierungsdaten zu erzeugen,
Bestimmen einer Häufigkeit (412) aller eindeutigen vorbestimmten komplementären Teile in den Sequenzierungsdaten für jede diskrete Einheit (200, 400), und
Zuordnen der Sequenzierungsdaten der biologischen Probe zu den Bildgebungsdaten der biologischen Probe (202) für eine bestimmte diskrete Einheit (200, 400) basierend auf der Häufigkeit der eindeutigen Label und der Häufigkeit der eindeutigen vorbestimmten komplementären Teile.

11. Verfahren nach Anspruch 10, wobei die Eigenschaften der Fluorophore (112, 406, 408, 504, 506, 508, 510, 512) die Anregungswellenlänge und Fluoreszenzwellenlänge umfassen.

12. Verfahren nach einem der Ansprüche 10 und 11, wobei die diskrete Einheit aus einer polymeren Verbindung, insbesondere einem Hydrogel, besteht.

13. Verfahren nach einem der Ansprüche 10 bis 12, wobei die diskreten Einheiten (200, 400) mittels eines Mikroskops abgebildet werden.

14. Verfahren nach einem der Ansprüche 10 bis 13, wobei die biologische Probe (202) mindestens eine Zelle umfasst.

## Revendications

1. Une pluralité de parties constitutives (100, 204, 300, 302, 402, 404, 500) d'un marqueur destiné à marquer des entités distinctes (200, 400), chaque partie constitutive comprenant :
une structure de support (102, 304),
au moins un premier oligonucléotide (104, 306) relié à la structure de support (102, 304),
au moins un deuxième oligonucléotide (106) au moins partiellement complémentaire d'une partie (108, 310) du premier oligonucléotide (104, 306), et
au moins une étiquette (112, 406, 408, 504, 506, 508, 510, 512) reliée au deuxième oligonucléotide (106), **caractérisée en ce que**
au moins une séquence de la partie complémentaire entre le premier oligonucléotide et le deuxième oligonucléotide est prédéterminée sur la base de l'étiquette particulière de la partie constitutive, dans laquelle la séquence prédéterminée de chaque partie constitutive est une séquence unique.

2. La partie constitutive selon la revendication 1, dans laquelle l'étiquette (112) comprend au moins un premier fluorophore (504).

3. La partie constitutive selon l'une des revendications précédentes, dans laquelle la structure de support (102, 304) est une microbille (102), une structure à base d'origami d'ADN (304) ou une nanorègle.

4. La partie constitutive selon l'une des revendications précédentes, dans laquelle l'étiquette (112) comprend au moins un second fluorophore (506).

5. La partie constitutive selon la revendication 4, dans laquelle le premier fluorophore (504) et le second fluorophore (506) diffèrent dans leurs propriétés optiques.

6. La partie constitutive selon l'une des revendications 2 à 5, dans laquelle le premier fluorophore (504) et/ou le second fluorophore (506) sont chacun reliés à des parties distinctes du deuxième oligonucléotide (106).

7. La partie constitutive selon l'une des revendications précédentes, dans laquelle la partie (108, 310) du premier oligonucléotide (104, 306) au moins partiellement complémentaire du deuxième oligonucléotide (106) et/ou la partie (110) du deuxième oligonucléotide (106) au moins partiellement complémentaire du premier oligonucléotide (104, 306) est clivable par rapport au premier oligonucléotide (104, 306) ou au deuxième oligonucléotide (106), respectivement.

8. La partie constitutive selon l'une des revendications précédentes, dans laquelle le premier fluorophore (104, 306) est relié au deuxième oligonucléotide (106) par un troisième oligonucléotide (514) au moins partiellement complémentaire d'une première partie du deuxième oligonucléotide (106).

9. La partie constitutive selon l'une des revendications 4 à 8, dans laquelle le second fluorophore (506) est relié au deuxième oligonucléotide (106) par un quatrième oligonucléotide (516) au moins partiellement complémentaire d'une seconde partie du deuxième oligonucléotide (106).

10. Procédé destiné à attribuer des données de séquençage à des données d'imagerie d'échantillons biologiques (202), le procédé comprenant les étapes suivantes :
fournir une pluralité d'entités distinctes (200, 400) comprenant chacune un échantillon biologique (202) et une pluralité de parties constitutives (100, 204, 300, 302, 402, 404, 500), selon l'une des revendications précédentes, les parties constitutives formant un marqueur de l'entité distincte (200, 400), dans lequel les parties constitutives sont regroupées en au moins un premier ensemble de parties constitutives et un second ensemble de parties constitutives, et, pour chacun des ensembles de parties constitutives, les parties constitutives comprennent une étiquette unique et une partie complémentaire prédéterminée unique entre le premier et le deuxième oligonucléotide,
imager les entités distinctes (200, 400) pour générer des données d'imagerie des échantillons biologiques correspondants (202) et des marqueurs,
déterminer une fréquence (410) de toutes les étiquettes uniques dans les données d'imagerie pour chaque entité distincte (200, 400),
désintégrer individuellement les entités distinctes (200, 400) pour libérer l'échantillon biologique correspondant (202) et les parties constitutives du marqueur,
séquencer individuellement les échantillons biologiques correspondants (202) et au moins les parties complémentaires prédéterminées correspondantes entre le premier oligonucléotide et le deuxième oligonucléotide des parties constitutives du marqueur afin de générer des données de séquençage,
déterminer une fréquence (412) de toutes les parties complémentaires prédéterminées uniques dans les données de séquençage pour chaque entité distincte (200, 400), et
attribuer, pour une entité distincte particulière (200, 400), les données de séquençage de l'échantillon biologique aux données d'imagerie de l'échantillon biologique (202) sur la base de la fréquence des étiquettes uniques et de la fréquence des parties complémentaires prédéterminées uniques.

11. Procédé selon la revendication 10, dans lequel les propriétés des fluorophores (112, 406, 408, 504, 506, 508, 510, 512) incluent la longueur d'onde d'excitation et la longueur d'onde de fluorescence.

12. Procédé selon l'une des revendications 10 et 11, dans lequel l'entité distincte est constituée d'un composé polymère, en particulier d'un hydrogel.

13. Procédé selon l'une des revendications 10 à 12, dans lequel les entités distinctes (200, 400) sont imagées à l'aide d'un microscope.

14. Procédé selon l'une des revendications 10 à 13, dans lequel l'échantillon biologique (202) comprend au moins une cellule.
